# EUROPEAN PATENT SPECIFICATION

(11) **EP 1 688 129 B1**
(45) Date of publication and mention of the grant of the patent: **08.07.2015**
(21) Application number: 06101197.9
(22) Date of filing: 02.02.2006
(51) Int. Cl.: A61K 9/00, A61K 8/00, A61Q 19/00

(54) **New therapeutic formulation**
Neue therapeutische Zusammensetzungen.
Nouvelle préparation thérapeutique

(30) Priority: 02.02.2005 SE 5002423
(43) Date of publication of application: 09.08.2006
(73) Proprietor: Omega Pharma Nordic AB, 164 07 Kista (SE)
(72) Inventor: Lodén, Marie, 170 77 Solna (SE); Åkerström, Ulf, 754 36 Uppsala (SE); Schwan, Emil, 753 27 Uppsala (SE)
(74) Representative: Holmberg, Martin Tor

(56) References cited:
- WO-A-92/05768
- WO-A-92/05769
- PATENT ABSTRACTS OF JAPAN vol. 1997, no. 10, 31 October 1997 (1997-10-31) & JP 09 169658 A (POLA CHEM IND INC), 30 June 1997 (1997-06-30)
- PATENT ABSTRACTS OF JAPAN vol. 007, no. 175 (C-179), 3 March 1983 (1983-03-03) & JP 58 083623 A (HISAMITSU SEIYAKU KK), 19 May 1983 (1983-05-19)
- PATENT ABSTRACTS OF JAPAN vol. 009, no. 021 (C-263), 29 January 1985 (1985-01-29) & JP 59 170011 A (POLA KASEI KOGYO KK), 26 September 1984 (1984-09-26)

## Description

### Background of invention

There are several examples of pharmaceuticals known to be degraded or altered by free radicals. Well-known inducers of free radicals in the skin are UV-irradiation and visible light, i.e. sunlight may decompose active substances and excipients in pharmaceuticals. The photodecomposition may occur on the skin surface in topical preparations, but also deeper within the skin or intracellularly after penetration of light into viable tissues. Photodecomposition decreases the effect of the drug, which may be stabilized using different additives and carriers. Examples of substances requiring stabilization are: Calcitriol ointment, approved for the treatment of psoriasis; Tretinon and benzoyl peroxide, approved for acne vulgaris; Amlodipine and Nifedipine, drugs used for hypertension and angina pectoris; Doxorubicin approved for treatment of a variety of cancers; Antimycotics such as terbinafine aftifine, sulbentine and cloxiquin; Antibiotics such as ofloxacin, amphotericin B, natamycin and nystatin; Minoxidil which is used for stimulation of hair growth; and the anti-inflammatory drug ketoprofen.

Ketoprofen is a non-steroidal anti-inflammatory drug (NSAID) marketed throughout the world in a variety of forms including tablets and topical formulations (patches, creams, ointments and gels) for the treatment of localized musculoskeletal diseases. Topical administration is used to improve the tolerability profile of NSAIDs, with regard to gastric and renal adverse effects. However, ketoprofen is photolabile and has often been implicated in photosensitivity reactions, including both phototoxic and photoallergic adverse effects. Reactive oxygen species and toxic decomposition products may be formed by this conversion. A major photoproduct from ketoprofen is 3-ethylbenzophenone, which is able to mediate photoperoxidation of linoleic acid. Protecting ketoprofen against sunlight in topical formulations provides a reduced risk for photosensitivity reactions. Gel formulations containing ketoprofen are available without prescriptions and are demonstrated useful for the treatment of pain and inflammatory conditions. However, many reports of photocontact dermatitis attributable to ketoprofen has been collected.

The photostability of a photosensitive pharmacologically active substance /ketoprofen in a topical formulation can increase by the addition of ultraviolet filters, such as ultraviolet light absorbers or ultraviolet scattering agents. Titanium dioxide is commonly used in packagings to protect against light transmission and as a white pigment in tablets and in colour cosmetics. The substance scatters and absorbs ultraviolet (UV) radiation and this effective attenuation of light has lead to its widespread use as physical UV filter in sunscreens

There are numerous references to technology related to prevent photosensitive compounds from undesired degradation. FR 2 804 024 refers to a pharmaceutical composition containing a non-steroid anti-inflammatory agent in combination with an ultraviolet light filtering composition such as a benzophenon derivative. However, chemical filters including benzophenon filters will be degraded following the exposure of sunlight, which potentially may generate reactive compounds that reacts with ketoprofen. Moreover, chemical UV-filters may in themselves produce adverse reactions in the skin due to their photosensitivity. It may also be of disadvantage that the filters not maintain their protective level. US 4,927,464 refers to a particulate material comprising non-calcinated titanium dioxide the particles of which are acicular in shape and have a coating comprising an oxide or hydrous oxide of aluminium and an oxide or hydrous oxide of silicon in a weight ratio of Al₂O₃:SiO₂ of at least 1.5 and not greater than 4.5. US 5,068,056 discloses an aqueous dispersion of acicular fine particle size titanium dioxide, water, and a dispersing agent consisting of polymers or copolymers of acrylic acid. The titanium dioxide particles, coated as described in US 4,927,464, are present in an amount making the dispersion substantially transparent to visible light.

There are a number of patents describing cosmetic sunscreen compositions containing titanium dioxide particles, for instance EP 0 559 319 B 1 referring to an oil-in-water emulsion comprising 0.5 to 30 % of a metallic oxide, which emulsion contains 10-60 % of an oil phase and at least 40 % of an aqueous phase. JP 9169658 discloses a pharmaceutical composition for external application to the skin, comprising a non-steroid anti-inflammatory agent and titanium dioxide without addressing any particular benefits from the titanium dioxide than its conventional sunscreen effects with reduced UV-induced erythema. WO 92/05769 refers to the usefulness of the specific enantiomer S(+) ketoprofen in topical sunscreen formulations. It is therefore a need for a versatile topical composition for photosensitive compounds based on titanium dioxide, which is formulated for maximal drug delivery by a purposeful selection of constituents that contribute to such an effect.

### Description of the invention.

It is an object of the present invention to provide a transcutaneous drug delivery system for photosensitive compounds that maximizes or improves intradermal levels of intact therapeutically active compound. It is another object of the invention to provide a titanium dioxide UV light absorber including transcutaneous drug delivery system that is adapted to maximize the efficacy of the titanium dioxide to perform its photostabilizing effect.

It is another object of the present invention to provide for a transcutaneous drug delivery system for photosensitive compounds that comprises an UV absorber and an antioxidant that minimizes any interactions between its constituents that lead to losses of the therapeutically active compound.

It is still another object of the invention a transcutaneous drug delivery system for photosensitive compounds wherein cosmetically acceptable constituents can be employed without losing any efficacy in reaching therapeutically effective levels of active compound following administration of said system.

It still yet another object of the present invention to provide transcutaneous drug delivery system for photosensitive compounds which is applicable with a great range of such compounds.

Further objects of the invention will be apparent from the following specification and its appended claims.

In its most general terms, the present invention relates to a transcutaneous drug delivery system, which comprises a transcutaneously administrable photosensitive therapeutically active agent with an absorbance below about 250 nm, a gelling agent, a solvent capable of evaporation following topical administration and capable of contributing to the transcutaneous transportation of active agent, and a cosmetically acceptable particulate titanium dioxide. The system may further comprise complementary constituents or additives of conventional nature, for example to increase the compliance.

The term "transcutaneously administrable" means that the agent can topically applied to surface of the skin in an excipient and localize to stratum corneum before reaching the site of activity. The term "photosensitive agent" will in the present context mean an agent following light exposure decompose and at least partially loose its original activity. The term "gelling agent" will in the present context mean an agent that swells following the contact with a hydrophilic solution, such as an aqueous solution and retains gel shape.

The presently invented delivery system is adapted to comply with the fact that for certain active agents following penetration of skin, the upper layers of corneum stratum provide no or limited protection of photodecomposition in the lower layers of corneum stratum. This phenomenon may be explained from that the tissue does not filter the wavelengths responsible for the decomposition of the specific agent. Consequently, there is a demand for a delivery system that minimizes loss of active compound on the skin surface and during the passage to the upper region of corneum stratum. Accordingly, the presently invented delivery system is purposefully assembled to meet such demands by admitting a protective rapid transfer of active compound to the upper regions of corneum stratum.

The titanium oxide used in the delivery system preferably comprises surface coated particles. The coating layer preferably comprises aluminium oxide and/or silicon oxide; typically it is a stable layer of aluminium and silica. Suitable amounts of titanium dioxide vary from 1 to 10% (w/w), preferably from 2 to 8% (w/w) and more preferably about 4% (w/w).

The gelling agent of the delivery system is selected so as avoid such interaction with the titanium dioxide particles that impair or compromise their shielding capacity. Also the delivery system must be suitably stable and compliant to apply to the skin. The choice of the gelling agent accordingly has a great influence on the final product properties. For a system, preferred according to the present invention comprising a water/alcoholic ketoprofen gel, a range of gelling agents is conceivable. However, when the gel comprises titanium oxide in dispersion, the gelling agent must be compatible with the titanium dioxide thickeners, as well as any dispersing agent present in the system. In accordance with the present invention, it has been found that non-ionic gelling agents, especially modified celluloses are suitable. Such agents have preferably are selected in a group of cellulose gelling agents with different rheologic properties, comprising hydroxyethyl cellulose, hydroxypropyl methylcellulose, hydroxyethyl ethylcellulose and hydroxymethyl cellulose. Hydroxypropyl methylcellulose (HPMC), such as Metocel, is an especially preferred cellulose derivative due to its excellent cosmetic properties.

The delivery system may also when regarded necessary include an antioxidant or mixtures of antioxidants having such characteristics that it does not interfere with its other components and the active ingredient The antioxidant suitably is selected from a group consisting of vitamin E or its derivatives, vitamin C or its derivatives BHT (butylated hydroxytoluene), BHA (butylated hydroxyanisole) or ubiquinone or its derivatives. An especially suitable antioxidant is found to be vitamin E acetate.

Suitable hydrophilic solvents are water, a mixture of water and alcohol or polyol, or an alcohol or polyol, or their mixtures. Suitable alcohols are ethanol, propanol, butanol and isopropyl alcohol, while suitable polyols are found among propylene glycol, butylene glycol or glycerol. According to one important aspect, the hydrophilic solvent is volatile in order to accomplish a supersaturated solution of active agent in order to trigger a rapid passage.

The delivery system is particularly useful to incorporate and administer photolabile non-steroidal anti-inflammatory drugs (NSAIDs), but numerous other substances liable to photodecomposition from exposure to sunlight will be equally useful. Suitable amounts of active agents vary from 0.5 to 2.5% (w/w) Especially, such agents which obtain limited protection from photodecomposition by the filtering effects of the upper layers of corneum stratum during the skin passage will fmd benefit from the inventive system. One particularly preferred active agent is ketoprofen.

According to a particularly preferred embodiment the drug delivery system is a gel preparation comprising the therapeutically active agent in a water-volatile alcohol mixture that provides stability to the active agent while being cosmetically acceptable. Preferably, the titanium dioxide is of a cosmetically acceptable brand that is coated with a layer of alumima and silica, such as Tioveil, AQ-N, and Uniquema. The solvent is capable of evaporation following topical administration and thereby contributing to the transcutaneous transportation of active agent by locally forming a supersaturated solution of active agent. For this purpose the system typically can comprise 30 % ethanol (w/w).

In a particularly preferred example the drug delivery system comprises 2.5 % (w/w) ketoprofen, 4 (w/w) % coated titanium dioxide (Tioveil, AQ-N, Uniquema), HPMC as a gelling agent, 30% (w/w) ethanol and vitamin E acetate as antioxidant.

The present invention also refers to a method of preparing systems for transcutaneous administration of photosensitive therapeutically active agent, which have been described in the foregoing. The method comprising the steps of: (a) dissolving the active agent in an alcohol optionally with water added; (b) adding a gelling agent to the solution of (a) and admitting the gelling agent to swell; (c) admixing the gel formed in step (b) with a dispersion of the titanium dioxide and water, so as to form a suitably homogenous product system. The gelling agent, typically an agent of polymeric nature, can either be powder form or a pre-wetted, or pre-swelled in a solvent. The recited preparation method allows for the use of many types and levels of gelling agents, as well as different amounts solvents for swelling. As previously mentioned, the gelling agent must be compatible with the titanium dioxide dispersion, as well as added in the right manner. Typically, in accordance with the present invention, this is performed by swelling the gelling agent and creating the gel before adding the dispersion, while taking into consideration the mixing force used to evenly mix the dispersion into the gel, as well as controlling the levels of titanium dioxide dispersion and alcohol during the method of preparation. By following the inventive method, the titanium dioxide dispersion can be correctly and optically dispersed without any introduction of lumps or grains, which may make the product useless.

### Detailed and exemplifying part of the invention

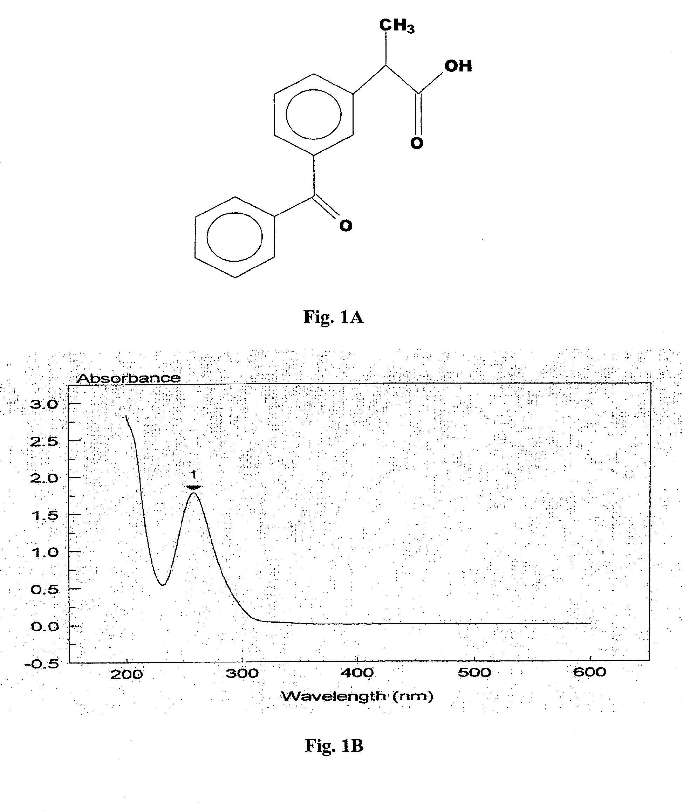

Figure 1 shows the chemical structure of ketoprofen (A) and its absorbance spectrum during the chemical analysis (B). 3-ethylbenzophenone has been reported to be the major photoproduct in neutral aqueous medium.

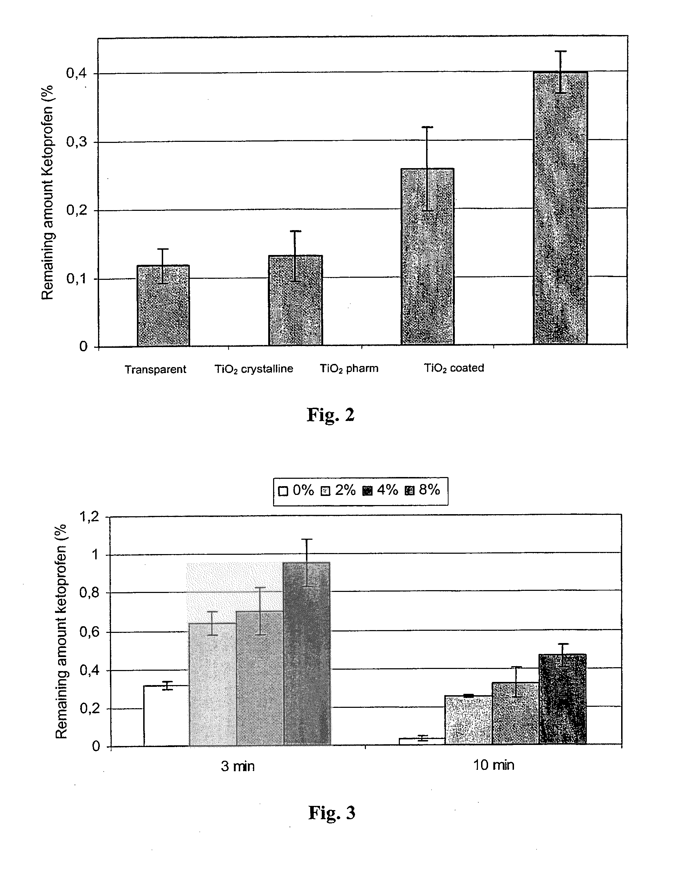

Figure 2. shows the remaining amount of ketoprofen in the transparent titanium dioxide-free carbomer gel (transparent) and of the hypromellose gel containing 4 % fine-crystalline titanium dioxide (TiO₂ crystalline), 4 % titanium dioxide pharmaceutical grade (TiO₂ pharm) and 4% coated titanium dioxide (TiO₂ coated) gels after 3 min of irradiation (11.7 J/ cm2 UVA and of 5.4 mJ/cm2 UVB ). N=5. Mean value ± S.D.
Figure 3 shows the amount of remaining ketoprofen with increasing concentration of coated titanium dioxide in the hypromellose gel and increasing dosages of UV-radiation. The initial concentration of ketoprofen was 2.5 %. Irradiation for 1 min corresponds to 3.9 J/cm2 UVA and 1.8 mJ/cm2 UVB. N=5 ± S.D.

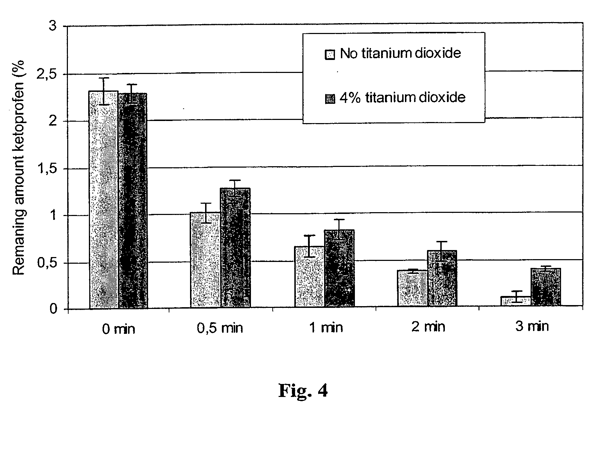

Figure 4 shows the amount of remaining ketoprofen (initial concentration 2.5 %) after increasing time of irradiation of a carbomer gel without titanium dioxide and a hypromellose gel containing 4 % coated titanium dioxide. Irradiation for 1 min corresponds to 3.9 J/cm2 of UVA and 1.8 mJ/cm2 of UVB. N=5 ± S.D.

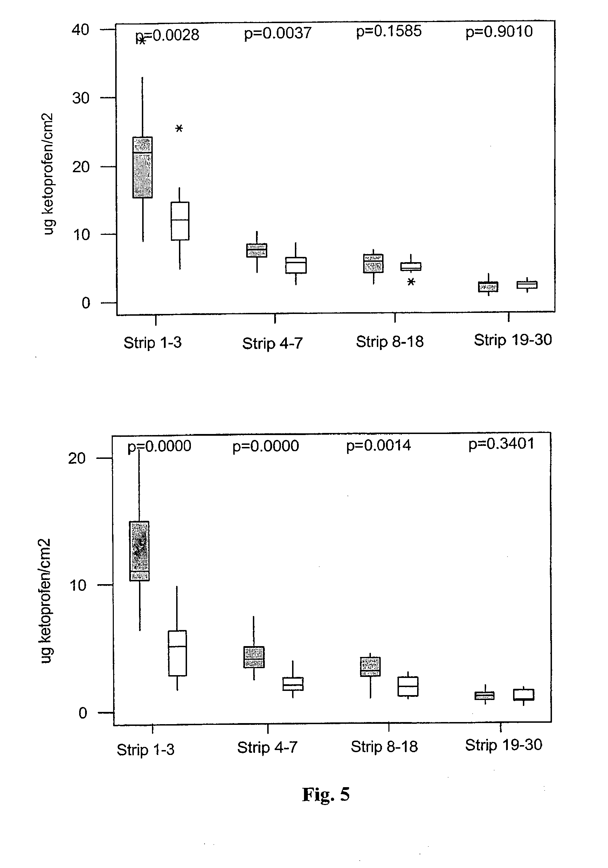

Figure 5 shows the amount of ketoprofen at different depths of stratum corneum in non-irradiated (A) and in UV-exposed areas (B). Grey boxes correspond to the photo-stabilized gel and white boxes the transparent gel. The number of tape strips denotes different compartments in the stratum corneum. P-values indicate statistical differences between the two formulations. N=15.

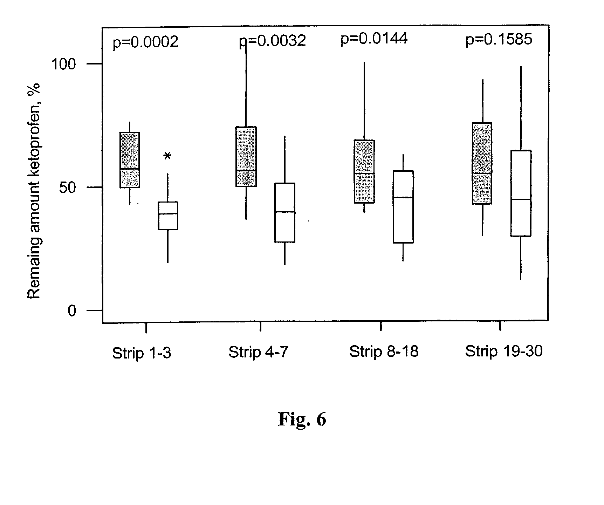

Figure 6 shows the amount of ketoprofen at different depths of stratum corneum in UV-exposed areas in percent of the amount in the non-exposed skin (i.e. Figure 4B in comparison to 4A). Grey boxes correspond to the photo-stabilized gel and white boxes the transparent gel. The number of tape strips denotes different compartments in the stratum corneum. P-values indicate statistical differences between the two formulations. N=15.

### Example of preparation

In order to obtain a suitably homogenous gel including ketoprofen and titanium dioxide the following production was employed to produce 200 kg product.

0.6 kg sodium hydroxide was added in 90 kg water in a vessel. Stirring was performed for approximately 5 minutes for complete dissolution. 60 kg of ethanol, 0.05 kg menthol and 5 kg ketoprofen were added into another vessel. Stirring was performed approximately 5 minutes for complete dissolution. 4 kg of hypromellose was added to the ethanolic solution, by stirring and mixing for approximately 5 minutes until a completely dispersed product was obtained. The basic aqueous solution was added during approximately 5 minutes to the ethanolic solution during stirring and mixing. The resulting solution was stirred and mixed for approximately 10 minutes until the gel was free from lumps and completely homogenous. In a separate vessel 20 kg titanium dioxide dispersion was added to 20 kg of water and stirred for approximately 5 minutes until the dispersion was homogenous. The diluted titanium dioxide dispersion was carefully transferred during approximately 10 minutes to the previously prepared water/ethanol gel. The fmal gel was stirred to homogeneity to especially ensure that all parts of the gel comprise titanium dioxide dispersion.

### Experimental conditions

Ketoprofen (2.5 %) in aqueous gels (pH 6-7) containing about 30 w/w % ethanol and different concentrations of titanium dioxide were studied. One transparent gel without titanium dioxide contained carbomer as thickener (Orudis®, Aventis Pharma), while those with titanium dioxide were white and used hypromellose as thickener due to its compatibility with the microparticles.

Three different qualities of titanium dioxide were studied. One extremely fine-crystalline quality with a surface area ≥300m²/g (Hombikat, Sigma-Aldrich), one pharmaceutical grade with surface area 9-11 m²/g (Titanium (IV) oxide, extra pure, Ph. Eur., BP, USP, Sigma-Aldrich), and one quality developed to be used in sunscreens which is surface-coated with a stable layer of alumina and silica to reduce the risks for photo-catalytic activities (Tioveil AQ-N, Uniquema).

Irradiation was performed with a SUPUVASUN 3000 lamp (Mutzhas, Germany). The lamp emits 65 mW/cm² in the UVA-range and 0.03 mW/cm² in the UVB-range at a distance of 50 cm, i.e. approximately 10 times higher UVA and 1/10 of UVB compared to natural sunlight at the earth's surface. Irradiation for 3 minutes will give a UVA-dose of 11.7 J/cm² and UVB-dose of 5.4 mJ/cm². Samples were also placed in direct sunlight between 12:00 noon and 2:00 pm in June at Uppsala, Sweden (latitude 59°N).

### In vitro experiments.

The gels were dispensed on frosted glass using a positive diplacement pipette and evenly spread by the fingertip. The dose applied (3 µl/cm²) is similar to the dose used for studies of sunscreen photostability in vitro (1-2 µl/cm²), see Stokes R et al., 1999, Int J Cosm Sci 21:341-351 and Berset G et al., 1996.. Int J Cosm Sci 18:167-177.After drying they were irradiated and assayed for ketoprofen.

### In vivo experiments.

Fifteen healthy caucasians (5 males 10 females, age 21-53 years; mean 28.1 ± 8.3 years) with no signs of skin diseases, sunburns, tattoos or scars in the treated area gave their informed consent to participate in the study. The study was conducted according to Declaration of Helsinki principles and approved by the ethics committee at Uppsala university.

Two gels were applied to the volar aspect of each forearm using a positive diplacement pipette and evenly spread by the fingertip ;the transparent gel without titanium dioxide and the white gel with (4 %) titanium dioxide. The rate of application (2 µl/cm2) reflects the dose applied to normal skin by healthy volunteers (1.7 µl/cm²), see Ivens UI et al.: 2001, Br J Dermatol 145:264-267. The gels were randomized to either the upper or the lower part of the forearm and each treated area was 1.8x2.5 cm. After 3 hours exposure to the formulations one of the arms (randomized) was irradiated for 3 min. The upper layers of the stratum corneum were then removed by successive stripping of the skin with tape. The adhesive tape was applied to each area with a uniform pressure and removed 30 times. Strips 1-3 were considered to represent unabsorbed drug on the skin surface. Strips 1-3, strips 4-7, strips 8-18 and strips 19-30 were collected in four separate vials.

### Chemical analysis.

Ketoprofen in the samples was extracted with methanol and injected into a reversed-phase high performance liquid chromatography (HPLC) according to a previously described method (M Lodén et al Int J Pharm, 2004, 284:23-30.

The chromatograph was equipped with a C18 (15 cm x 3 mm, 4 µm particles) column with UV-detector set at 259 nm, where the molecule had its extinction maximum, Figure 1b. The mobile phase was isocratic acetonitrile - 0.01M potassium phosphate pH adjusted to 2.9 with phosphoric acid (40:60 v/v) with a flow rate of 0.5 ml/min. The peak areas of the decomposition products were calculated but their identity were not determined.

### Calculations and statistics.

Each bar represents the mean value ± standard deviation (S.D.) of the *in vitro* data, whereas the *in vivo* data are presented using box-plots. The box is defined by the upper and lower quartiles and with the median marked by a subdivision of the box. The whiskers have a maximum length in the terms of the interquartile range and outliers are shown (Minitab Statistical Software, Minitab Inc., PA, USA). The non-parametric Wilcoxon signed rank test on paired data was used when comparing the results and p<0.05 was adopted as the level of significance.

### Results

Ketoprofen is photolabile and was substantially degraded *in vitro* after 3 min UV-irradiation (Fig 2) in the solar simulator (11.7 J/cm² of UVA and 5.4 mJ/cm² of UVB). Addition of 4 % titanium dioxide to the gel influenced the decomposition of ketoprofen (Fig 2). Extremely fine-crystalline titanium dioxide gave almost no protection, whereas the other quality grades significantly improved the stability, with the most pronounced effects observed from the surface-coated microparticles (Fig 2).

The protection by the surface-coated titanium dioxide was dependent on the concentration of the microparticles and 2 %, 4 % and 8 % increased the amount of remaining ketoprofen accordingly (Fig 3). The decomposition was also found to be related to the length of UV-irradiation, with larger decomposition with increasing UV-dose (Fig 3 and Fig 4). After exposure to ordinary sunlight for 2 hr the transparent gel contained approximately the same amount of ketoprofen as after irradiation for 10 min in the solar simulator (i.e. approximately 2 % of the applied amount), whereas the gel containing 4 % coated titanium dioxide contained approximately 30 % of the applied amount after sunlight exposure and 15 % after exposure to 10 minutes to UV-irradiation in the solar simulator. Table 1.

Ketoprofen was absorbed into the skin from the gels during the 3-h treatment period (Fig 5A). Higher levels of ketoprofen were found on the surface (strips 1-3) and in strips 4-7 after treatment with the titanium dioxide containing gel than after treatment with the transparent gel, whereas in the deeper layers of the stratum corneum similar contents of ketoprofen were found (Fig 5A).

After irradiation of the skin the amount of ketoprofen was decreased to about half of that in the corresponding non-irradiated area, also demonstrating the photolability of the substance (c.f. Fig 5A and 5B). Titanium dioxide protected ketoprofen and 30-120 % higher levels were found on the surface and in the deeper parts of the stratum corneum than in the areas treated with the transparent gel (Fig 5B). However, since higher levels of ketoprofen were found before irradiation, the percentages remaining after irradiation in relation to the non-irradiated amounts are shown in Fig 6. The results show that also after this adjustment the surface-coated titanium dioxide in the gel protected ketoprofen and increased its photostability (Fig 6).

The decomposition products were not identified and quantified. The largest UV-absorbing peak was eluted immediately after ketoprofen in the chromatograms and was below 10% of the ketoprofen peak. Its area-% was significantly lower in the skin treated with the titanium dioxide gel than in the skin treated with the transparent gel in all analysed stratum corneum compartments (data not shown).

It is also noted that HPMC based gels confer superior product stability and compliance when compared to carbomer based gels. The carbomer gels became unacceptably grainy and thick, and agglomerates were formed. These finds were unexpected, since the titanium dioxide particles were dispersed in a polyacrylate and compatibility with polyacylate derivatives was anticipated.

**Table 1. The influence of coated titanium dioxide concentration on the remaining amount of ketoprofen after exposure to ordinary sunlight between 12 am and 2 pm during a summer day in Sweden. The initial concentration of ketoprofen in the gel was 2.5 %. N=3 ± S.D.**

| Concentration of titanium dioxide (%) | Remaining concentration of ketoprofen (%) |
|---|---|
| 0 | 0.05 ± 0.02 |
| 2 | 0.24 ± 0.03 |
| 4 | 0.71 ± 0.12 |

## Claims

1. A transcutaneous drug delivery system comprising:
a. 0.5 to 5 % (w/w) of a transcutaneously administrable photosensitive non-steroid anti-inflammatory drug (NSAID;
b. a a non-ionic gelling agent selected from the group of cellulose gelling agents;
c. a hydrophilic solvent;
d. a cosmetically acceptable particulate titanium dioxide.

2. A system according to claim 1, wherein said cellulose gelling agent is a hydroxypropyl methylcellulose (HPMC).

3. A system according to claim 1, further comprising an antioxidant.

4. A system according to claim 3, wherein the antioxidant is selected from a group consisting of vitamin E or its derivatives, vitamin C or its derivatives BHT (butylated hydroxytoluene), BHA (butylated hydroxyanisole) or ubiquinone or its derivatives.

5. A system according to claim 1, wherein the particulate titanium oxide comprises coated particles.

6. A system according to claim 5, wherein the coating comprises aluminium oxide and/or silicon oxide.

7. A system according to claim 1, wherein the hydrophilic solvent is water and/or an alcohol or polyol, wherein the alcohol or polyol is selected from a group that consists of ethanol, propanol, butanol and isopropyl alcohol, propylene glycol, butylene glycol or glycerol.

8. A system according to claim 7, wherein the solvent is capable of evaporation following topical administration and is capable and of contributing to the transcutaneous transportation of active agent by locally forming a supersaturated solution of active agent.

9. A system according to claim 1, wherein the NSAID is ketoprofen.

10. A system according to claim 9, comprising 2.5 % (w/w) ketoprofen.

11. A system according to claim 7, comprising 30% (w/w) ethanol.

12. A system according to claim 1 comprising 1 to 10 % (w/w) titanium dioxide.

13. A system according to any previous claim comprising 2.5 % (w/w) ketoprofen, 4 % (w/w) coated titanium dioxide, 2 % (w/w) HPMC, 30% (w/w) ethanol and vitamin E.

14. A method of preparing a system for transcutaneous administration of photosensitive therapeutically active agent according to any of claims 1 to 13, comprising the steps of: (a) dissolving the active agent in an alcohol optionally with water added; (b) adding a gelling agent to the solution of (a) and admitting the gelling agent to swell; (c) admixing the gel formed in step (b) with a dispersion of the titanium dioxide and water, so as to form a suitably homogenous product system.

15. A method according to claim 14, wherein the gelling agent is a non-ionic cellulose derivative selected among hydroxyethyl cellulose, hydroxypropyl methylcellulose, and hydroxyethyl ethylcellulose and hydroxymethyl cellulose.

16. A method according to claim 14, wherein the alcohol is ethanol.

17. A method according to claim 14, wherein the active agent is an alcohol soluble non-steroid anti-inflammatory drug.

18. A method according to claim 17, wherein the active agent is ketoprofen.

## Patentansprüche

1. Transkutanes Wirkstoffabgabesystem umfassend:
(a) 0,5 bis 5 % (w/w) von einem transkutan verabreichbaren lichtempfindlichen nicht-steroidalen Antirheumatikum (NSAID);
(b) nicht-ionisches Geliermittel ausgewählt aus der Gruppe von Cellulose-Geliermittel;
(c) hydrophiles Lösungsmittel;
(d) einen kosmetisch verträgliches teilchenförmiges Titandioxid.

2. System nach Anspruch 1, wobei das Cellulose-Geliermittel eine Hydroxypropylmethylcellulose (HPMC) ist.

3. System nach Anspruch 1, weiterhin umfassend ein Antioxidans.

4. System nach Anspruch 3, wobei das Antioxidans ausgewählt ist aus einer Gruppe bestehend aus Vitamin E oder seinen Derivaten, Vitamin C oder seinen Derivaten, BHT (butyliertes hydroxytoluol), BHA (butyliertes hydroxyanisol) oder Ubichinon oder seinen Derivaten.

5. System nach Anspruch 1, wobei das teilchenförmige Titanoxid beschichtete Partikel umfasst.

6. System nach Anspruch 5, wobei die Beschichtung Aluminiumoxid und/oder Siliziumoxid umfasst.

7. System nach Anspruch 1, wobei das hydrophile Lösungsmittel Wasser und/oder ein Alkohol oder Polyol ist, wobei der Alkohol oder Polyol ausgewählt ist aus einer Gruppe, die aus Ethanol, Propanol, Butanol und Isopropylalkohol, Propylenglykol, Butylenglykol oder Glycerin besteht.

8. System nach Anspruch 7, wobei das Lösungsmittel zur Verdunstung fähig ist nach topischer Anwendung und fähig ist an dem transkutanen Transport des aktiven Mittels durch lokale Bildung einer übersättigten Lösung des aktiven Mittels mitzuwirken.

9. System nach Anspruch 1, wobei das NSAID Ketoprofen ist.

10. System nach Anspruch 9, umfassend 2,5% (w/w) Ketoprofen.

11. System nach Anspruch 7, umfassend 30% (w/w) Ethanol.

12. System nach Anspruch 1, umfassend 1 bis 10% (w/w) Titandioxid.

13. System nach einem der vorhergehenden Ansprüche, umfassend 2,5% (w/w) Ketoprofen, 4% (w/w) beschichtetes Titandioxid, 2% (w/w) HPMC, 30% (/w) Ethanol und Vitamin E.

14. Verfahren zur Herstellung eines Systems zur transkutanen Verabreichung von lichtempfindlich therapeutisch aktivem Wirkstoff nach einem der Ansprüche 1 bis 13, umfassend die Schritte:
(a) Lösen des Wirkstoffs in einem Alkohol, optional mit Wasser zugegeben;
(b) Zugabe eines Geliermittel zu der Lösung von (a) und Zulassen des Geliermittels zu quellen;
(c) Zumischen des Gels gebildet Schritt (b) mit einer Dispersion aus dem Titandioxid und Wasser, um ein geeignetes homogenes Produkt-System zu bilden.

15. Verfahren nach Anspruch 14, wobei das Geliermittel ein nicht-ionisches Cellulose-Derivat ausgewählt ist unter Hydroxyethylcellulose, Hydroxypropylmethylcellulose, und Hydroxyethylethylcellulose und Hydroxymethylcellulose.

16. Verfahren nach Anspruch 14, wobei der Alkohol Ethanol ist.

17. Verfahren nach Anspruch 14, wobei das aktive Mittel ein alkohollöslicher nicht-steroidaler entzündungshemmender Wirkstoff ist.

18. Verfahren nach Anspruch 17, wobei das aktive Mittel Ketoprofen ist.

## Revendications

1. Système d'administration de médicament par voie transcutanée, comprenant :
a. 0,5 à 5 % (en p/p) d'un médicament anti-inflammatoire non stéroïdien (NSAID) photosensible administrable par voie sous-cutanée ;
b. un agent gélifiant non ionique choisi dans le groupe des agents gélifiants cellulosiques ;
c. un solvant hydrophile ; et
d. un dioxyde de titane particulaire acceptable au plan cosmétique.

2. Système selon la revendication 1, dans lequel ledit agent gélifiant cellulosique est une hydroxypropylméthylcellulose (HPMC).

3. Système selon la revendication 1, comprenant en outre un antioxydant.

4. Système selon la revendication 3, dans lequel l'antioxydant est choisi dans un groupe constitué de la vitamine E ou de ses dérivés, de la vitamine C ou de ses dérivés, du BHT (hydroxytoluène butylé), du BHA (hydroxyanisole butylé) ou de l'ubiquinone ou de ses dérivés.

5. Système selon la revendication 1, dans lequel l'oxyde de titane particulaire comprend des particules enrobées.

6. Système selon la revendication 5, dans lequel l'enrobage comprend de l'oxyde d'aluminium et/ou de l'oxyde de silicium.

7. Système selon la revendication 1, dans lequel le solvant hydrophile est l'eau et/ou un alcool ou un polyol, dans lequel l'alcool ou le polyol est choisi dans un groupe constitué de l'éthanol, du propanol, du butanol et de l'alcool isopropylique, du propylèneglycol, du butylèneglycol ou du glycérol.

8. Système selon la revendication 7, dans lequel le solvant est capable de s'évaporer après administration topique et est capable de contribuer au transport transcutané de l'agent actif en formant localement une solution sursaturée d'agent actif.

9. Système selon la revendication 1, dans lequel le NSAID est le cétoprofène.

10. Système selon la revendication 9, comprenant 2,5 % (en p/p) de cétoprofène.

11. Système selon la revendication 7, comprenant 30 % (en p/p) d'éthanol.

12. Système selon la revendication 1, comprenant 1 à 10 % (en p/p) de dioxyde de titane.

13. Système selon l'une quelconque des revendications précédentes, comprenant 2,5 % (en p/p) de cétoprofène, 4 % (en p/p) de dioxyde de titane enrobé, 2 % (en p/p) de HPMC, 30 % (en p/p) d'éthanol et de la vitamine E.

14. Procédé de préparation d'un système d'administration transcutanée d'un agent photosensible actif au plan thérapeutique selon l'une quelconque des revendications 1 à 13, comprenant les étapes consistant à (a) dissoudre l'agent actif dans un alcool éventuellement additionné d'eau ; (b) ajouter un agent gélifiant à la solution de (a) et laisser l'agent gélifiant gonfler ; (c) et mélanger le gel formé à l'étape (b) avec une dispersion du dioxyde de titane et d'eau de manière à former un système de produit convenablement homogène.

15. Procédé selon la revendication 14, dans lequel l'agent gélifiant est un dérivé de cellulose non ionique choisi parmi l'hydroxyéthylcellulose, l'hydroxypropylméthylcellulose, l'hydroxyéthyl-éthylcellulose et l'hydroxyméthylcellulose.

16. Procédé selon la revendication 14, dans lequel l'alcool est l'éthanol.

17. Procédé selon la revendication 14, dans lequel l'agent actif est un médicament anti-inflammatoire non stéroïdien soluble dans les alcools.

18. Procédé selon la revendication 17, dans lequel l'agent actif est le cétoprofène.
